# FASCICULE DE BREVET EUROPEEN

(11) **EP 1 351 977 B1**
(45) Date de publication et mention de la délivrance du brevet: **11.08.2004**
(21) Numéro de dépôt: 02710946.1
(22) Date de dépôt: 07.01.2002
(51) Int. Cl.: C07K 7/02, C07K 14/24, A61K 38/08, C12P 21/04, A01N 63/02, A61P 31/00

(54) **PSEUDOPEPTIDES ANTIMICROBIENS**
ANTIMIKROBIELLE PSEUDOPEPTIDE
ANTIMICROBIAL PSEUDOPEPTIDES

(30) Priorité: 12.01.2001 FR 0100415
(43) Date de publication de la demande: 15.10.2003
(73) Titulaire: INSTITUT NATIONAL DE LA RECHERCHE AGRONOMIQUE (INRA), 75007 Paris Cédex 07 (FR)
(72) Inventeur: THALER, Jacques, F-34980 Saint Clément de Rivière (FR); GIVAUDAN, Alain, F-34270 Saint Mathieu de Treviers (FR); LATORSE, Marie-Pascale, F-69490 Saint Romain de Popey (FR)
(74) Mandataire: Callon de Lamarck, Jean-Robert
(86) Numéro de dépôt international: PCT/FR2002/000032
(87) Numéro de publication internationale: WO 2002/055545

(56) Documents cités:
- WO-A-99/64449
- US-A- 5 827 872
- LI J ET AL.: "SYNTHESIS AND ANTISTAPHYLOCOCCAL ACTIVITY OF NEMATOPHIN AND ITS ANALOGS" BIOORGANIC & MEDICINAL CHEMISTRY LETTERS, OXFORD, GB, vol. 7, no. 10, 20 mai 1997 (1997-05-20), pages 1349-1352, XP004136332 ISSN: 0960-894X

## Description

La présente invention concerne des molécules antimicrobiennes de structure pseudopeptidique. En particulier, ces molécules sont isolées à partir de bactéries du genre *Xenorhabdus*. Elles trouvent notamment leurs applications en protection des cultures, en tant que pesticides permettant de lutter contre les maladies des plantes causées par des agents microbiens, et dans les domaines pharmaceutiques et vétérinaires en tant qu'agents antimicrobiens permettant de lutter contre les maladies humaines et animales d'origine microbienne.

En santé végétale comme en santé animale et humaine, il existe un besoin permanent d'obtention de nouvelles molécules permettant de lutter contre les maladies d'origine microbienne, notamment d'origine fongique, bactérienne ou virale. La protection des cultures contre ces maladies passe essentiellement par la pulvérisation de pesticides de synthèse sur lesdites cultures. Cependant, il existe aujourd'hui un besoin grandissant d'utiliser des composés de moins en moins toxiques pour l'homme et pour l'environnement, et de trouver des molécules nouvelles qui pourront remplacer celles vis-à-vis desquelles les microorganismes phytopathogènes sont devenus résistants. D'autre part, en santé humaine, il est également nécessaire de trouver de nouvelles molécules antimicrobiennes permettent de lutter contre les organismes pathogènes microbiens devenus résistants aux traitements existants, notamment les germes nosocomiaux multirésistants aux antibiotiques. De tels composés sont trouvés notamment parmi les molécules d'origine naturelle présentes chez les organismes vivants. En effet, la plupart des organismes vivants ont développé un arsenal de molécules de défense leur permettant de faire face aux diverses agressions qu'ils subissent dans leur environnement, en particulier de la part d'autres organismes vivants. Ces molécules sont directement utilisables en tant que principe actif. Cette démarche est également applicable dans le domaine pharmaceutique.

Le problème technique de la présente invention consiste donc à isoler de nouveaux composés antimicrobiens actifs, en particulier antifongiques et/ou antibactériens et/ou antiviraux, parmi les molécules naturelles produites par des organismes vivants. De tels composés trouveront en particulier des applications dans les domaines de la protection des cultures, mais également dans ceux de la santé et de la nutrition humaines et animales.

Parmi les composés qu'il est possible de trouver dans les organismes vivants, on trouve d'une part, des composés de nature protéique, générés à partir du code génétique et constitués d'un assemblage d'acides aminés, et d'autre part les autres composés, définis par défaut comme étant de nature non-protéique, générés à partir des éléments nutritifs desdits organismes et de l'activité catalytique de certains des composés de nature protéique, les enzymes.

Un certain nombre de composés pesticides d'origine naturelle a déjà été isolé à partir d'organismes vivants. Parmi les composés de nature protéique, on peut citer par exemple les toxines insecticides issues de la bactérie *Bacillus thuringiensis*, ainsi que les peptides antibactériens et antifongiques issus des invertébrés, en particulier des insectes. L'inconvénient majeur de ces molécules est leur nature protéique, synonyme d'un poids moléculaire important, lequel poids moléculaire rend difficile leur utilisation dans des compositions agrochimiques destinées à être pulvérisées sur les plantes. La meilleure protection apportée par de telles molécules de nature protéique est essentiellement obtenue à travers la transformation génétique des plantes à protéger avec un gène codant pour une de ces molécules, de manière à ce que lesdites plantes expriment directement dans leurs tissus lesdites molécules.

Un certain nombre de composés de nature protéique de faible poids moléculaire, des peptides, a également été soit isolé, soit synthétisé de manière artificielle. Les peptides sont des composés de nature protéique constitués d'un faible nombre d'acides aminés, généralement inférieur à 50. A ce titre, de nombreux peptides anti-microbiens participant au système de défense naturelle des organismes ont été isolés, notamment à partir de plantes, d'invertébrés, en particulier des insectes, mais aussi à partir de mammifères. Certains peptides possédant des propriétés antibiotiques ont également été isolés à partir de microorganismes. De tels peptides possèdent généralement un très faible nombre d'acides aminés, souvent inférieur à 10. A titre d'exemple, on peut citer la Majusculamide C décrite dans la demande de brevet US 4,342,751. Des bibliothèques de peptides synthétisés artificiellement ont également été créées par la méthode de la chimie combinatoire. Un exemple de peptides obtenus par ce procédé est par exemple décrit dans la demande de brevet WO 98/26793.

Il arrive également que certains peptides soient cycliques. Cette caractéristique peut être soit naturelle, comme par exemple pour les peptides décrits dans la demande de brevet WO 99/59412, soit artificielle, comme par exemple pour les peptides décrits dans les demandes de brevets WO 97/05163 et WO 00/20441.

Enfin, on connaît également des composés actifs isolés à partir d'organismes vivants de nature pseudopeptidique. Des composés de nature pseudopeptidique sont des composés comprenant une structure peptique constituée par un assemblage d'acides aminés, et une structure de nature non-peptidique. Un exemple de composé pseudo-peptidique isolé à partir d'une souche de la bactérie *Bacillus subtilis* est par exemple décrit dans la demande de brevet WO 98/50422.

Les bactéries des genres *Xenorhabdus* et *Photorhabdus* sont connues pour être des sources de composés actifs. Ces bactéries vivent en relation symbiotique avec des nématodes de la famille des *Steinernematidae* et des *Heterorhabditidae*, et ces nématodes constituent notamment des vecteurs d'infestation des insectes pour ces bactéries. En particulier, ces bactéries entomopathogènes ont permis l'isolement de protéines toxiques vis-à-vis des insectes. Un complexe protéique insecticide à été isolé à partir des bactéries du genre *Photorhabdus*. Ce complexe protéique est par exemple décrit dans les demandes de brevets WO 97/17432, WO 98/08932, et dans Bowen et al. (1998, Science 280, 2129-2132). Des polypeptides insecticides ont également été isolés à partir des bactéries du genre *Xenorhabdus.* De tels polypeptides sont par exemple décrits dans les demandes de brevets WO 95/00647, WO 98/08338, WO 98/50427, WO 99/03328, WO 99/54472 ou WO 00/30453.

Ces deux genres bactériens sont également connus pour être des sources de produits antimicrobiens, et ont notamment permis l'isolement de plusieurs familles de composés antibiotiques. Parmi ces familles, les bactéries du genre *Xenorhabdus* ont permis l'isolement des Xenorhabdines décrites dans la demande de brevet WO 84/01775 et dans McInerney et al. (1991, J. Nat. Prod. 54, 774-784), des Xenorxides décrites dans la demande de brevet WO 96/32396, des Xenocoumacines décrites dans la demande de brevet WO 86/01509 et dans McInerney et al. (1991, J. Nat. Prod. 54, 785-795), ainsi que des composés dérivés de l'indole, les nématophines, tels que décrits dans le brevet US 5,569,668 et dans Sundar and Chang (1993, J. Gen. Microbiol. 139, 3139-3148), Li et al. (1996, J. Nat. Prod. 59, 1157-1158), et Li et al. (1997, Can. J. Microbiol. 43, 770-773). Les bactéries du genre *Photorhabdus* ont permis l'isolement de composés antimicrobiens de la famille des Hydroxystilbenes tels que décrits dans Richardson et al. (1988, Appl. Environ. Microbiol. 54, 1602-1605), Sundar and Chang (1992, Antimicrob. Agent Chemother. 36,2645-2651), Li et al. (1995, Appl. Environ. Microbiol. 61, 4329-4333), et Hu et al. (1998, , Can. J. Microbiol. 44, 1072-1077), ainsi que des composés de la famille des polykétides ou antraquinones tels que décrits dans Li et al. (1995, Appl. Environ. Microbiol. 61, 4329-4333) et Hu et al. (1998, , Can. J. Microbiol. 44, 1072-1077).

Le seul composé antimicrobien de nature protéique isolé à partir de ces bactéries est la Xenorhabdicine. Ce composé décrit dans Thaler et al. (1995, Appl. Environ. Microbiol. 61, 2049-2052) présente une forte homologie avec une queue de phage.

### Description

La présente invention concerne un composé de nature pseudopeptidique possédant des propriétés antimicrobiennes et isolé à partir d'une bactérie du genre *Xenorhabdus*.

Par composé de nature pseudopeptidique, on entend selon l'invention un composé dont la structure comprend une portion de nature peptidique, constituée de résidus acides aminés, et une portion de nature non peptidique.

Par propriétés antimicrobiennes, on entend des propriétés antibactériennes et/ou antifongiques et/ou antivirales.

Par propriétés antibactériennes, antifongiques et antivirales, on entend non seulement la propriété caractérisée respectivement par la destruction mortelle des bactéries, champignons, et virus, mais également la propriété bactériostatique, fongistatique, et virostatique, caractérisée par l'inhibition de la croissance desdits bactéries, champignons, et virus.
Le composé pseudo-peptidique selon l'invention est caractérisé en ce qu'il est de formule (I) 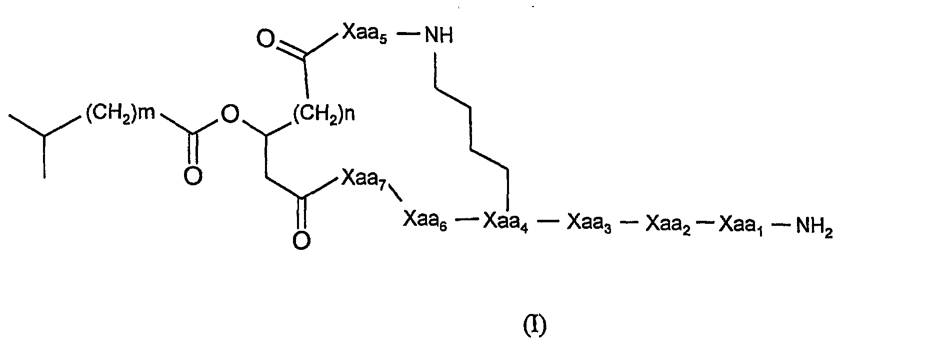 dans laquelle:
- Xaa (1 à 6) est un résidu acide aminé basique, de préférence la Lysine ou l'Arginine
- Xaa 7 est la glycine ou un résidu acide aminé à chaîne latérale aliphatique
- m = 1 ou 2 et n = 2 ou 3

Selon un mode particulier de réalisation de l'invention, les résidus Xaa 1 à 6 sont des résidus Lysine.

Selon un autre mode particulier de réalisation de l'invention, les résidus Xaa 1, 2, 3, 4, et 5 sont des résidus Lysine, et le résidu Xaa 6 est un résidu Arginine.

Selon un autre mode particulier de réalisation de l'invention, le résidu Xaa 7 est un résidu Glycine.

Selon un mode particulier de réalisation de l'invention, m est égal à 1 et n est égal à 3.

Selon un autre mode particulier de réalisation de l'invention, m est égal à 2 et n est égal à 2.

Le composé de formule (I) possède une activité antifongique. En particulier, il est actif vis-à-vis des champignons phytopathogènes. Plus particulièrement, il est actif sur les champignons du genre *Botrytis,* en particulier *B. cinerea*, du genre *Piricularia*, en particulier *P. oryzae*, du genre *Helminthosporium,* en particulier *H. teres*, du genre *Fusarium*, en particulier *F. culmorum*, du genre *Septoria,* en particulier *S*. *tritici*, du genre *Alternaria,* en particulier *A. brassicae*, du genre *Rhizoctonia,* en particulier *R. solani*, du genre *Phytophtora*, et du genre *Cladosporium*. Il est également actif vis-à-vis des champignons pathogènes humains. Plus particulièrement, il est actif sur les champignons du genre *Candida*.

Le composé de formule (I) possède également une activité antibactérienne. En particulier, il est actif vis-à-vis des bactéries à Gram positif et des bactéries à Gram négatif, qu'elles soient non-pathogènes, pathogènes de l'Homme ou des animaux, ou encore phytopathogènes. Plus particulièrement, il est actif vis-à-vis des bactéries du genre *Escherichia,* en particulier *E. coli*, du genre *Salmonella,* du genre *Klebsiella*, en particulier du *K. pneumoniae*, du genre *Proteus,* en particulier, *P. vulgaris*, du genre *Serratia,* en particulier *S. entomophila*, du genre *Staphylococcus*, en particulier *S. epidermis*, du genre *Steptococcus,* du genre *Pseudomonas,* en particulier *P. fluorescens*, du genre *Bacillus,* en particulier *B. megaterium*.

Le composé de formule (I) est également actif vis-à-vis des bactéries nosocomiales, notamment les bactéries du genre *Staphylococcus,* en particulier *S. aureus*, du genre *Pseudomonas,* en particulier *P. aeruginosa*, du genre *Enterococcus,* du genre *Enterobacter,* en particulier *E. aerogenes*, ou du genre *Stenotrophomonas,* en particulier *S. maltophila*.

Le composé de formule (I) possède également une activité antivirale. En particulier, il est actif vis-à-vis des virus encapsidés. Plus particulièrement, il est actif vis-à-vis des phages, en particulier le phage lambda.

La présente invention concerne également un procédé de production d'un composé pseudopeptidique antimicrobien selon l'invention, caractérisé en ce qu'il comprend les étapes de:
(a) mise en culture d'une bactérie du genre *Xenorhabdus* jusqu'à atteinte de la phase stationnaire de culture
(b) isolement du surnageant de culture
(c) séparation des composés contenus dans le surnageant par passage dudit surnageant dans au moins une colonne de chromatographie
(d) isolement sélectif d'au moins un composé selon l'invention par isolement des fractions d'élution correspondant aux pics de chromatographie

De manière préférée, la bactérie mise en culture à l'étape (a) est une bactérie de l'espèce *Xenorhabdus nematophilus* (également nommée *Xenorhabdus nematophila*; voir Euzeby and Boemare, 2000, Int. J. Syst. Evol. Microbiol. 50(4), 1691-1692).

Selon un mode particulier de réalisation de l'invention, l'isolement du surnageant de culture se fait par centrifugation de ladite culture.

Selon un autre mode particulier de réalisation de l'invention, l'isolement du surnageant de culture se fait par filtration de ladite culture.

La présente invention concerne également des compositions agrochimiques antimicrobiennes comprenant comme matière active au moins une quantité efficace d'un composé pseudopeptidique de formule (I).

Par composition agrochimique, on entend selon l'invention une composition applicable de manière préventive ou curative sur des végétaux cultivés ou destinés à être cultivés, ainsi que sur les surfaces sur lesquelles ces végétaux sont ou doivent être cultivés, afin de les protéger contre des maladies d'origine microbienne, quel que soit le stade de développement desdits végétaux. Une quantité efficace de composé correspond à une quantité de composé permettant de détruire ou d'inhiber la croissance des agents microbiens.

Les compositions agrochimiques antimicrobiennes selon l'invention comprennent un composé de formule (I) ou un de ses sels acceptables en agriculture ou un complexe métallique ou métalloïdique de ce composé, en association avec un support solide ou liquide, acceptable en agriculture et/ou un agent tensioactif également acceptable en agriculture. En particulier sont utilisables les supports inertes usuels et les agents tensioactifs usuels. Ces compositions recouvrent non seulement les compositions prêtes à être appliquées sur une plante ou une semence à traiter au moyen d'un dispositif adapté, tel qu'un dispositif de pulvérisation ou de poudrage, mais également les compositions concentrées commerciales qui doivent être diluées avant application sur la culture.

Les compositions antimicrobiennes selon l'invention peuvent contenir aussi de nombreux autres ingrédients tels que, par exemple, des colloïdes protecteurs, des adhésifs, des épaississants, des agents thixotropes, des agents de pénétration, des stabilisants, des séquestrants. Plus généralement, les matières actives peuvent être combinées à tous les additifs solides ou liquides correspondant aux techniques habituelles de la mise en formulation.

Par le terme "support", on désigne selon la présente invention une matière organique ou minérale, naturelle ou synthétique, avec laquelle la matière active est combinée pour faciliter son application sur les parties de la plante. Ce support est donc généralement inerte et il doit être acceptable en agriculture. Le support peut être solide (par exemple des argiles, silicates naturels ou synthétiques, silice, résines, cires, engrais solides) ou liquide (par exemple de l'eau, des alcools, notamment le butanol).

L'agent tensioactif peut être un agent émulsionnant, dispersant ou mouillant de type ionique ou non ionique ou un mélange de tels agents tensioactifs. On peut citer par exemple des sels d'acides polyacryliques, des sels d'acides lignosulfoniques, des sels d'acides phénolsulfoniques ou naphtalènesulfoniques, des polycondensats d'oxyde d'éthylène sur des alcools gras ou sur des acides gras ou sur des amines grasses, des phénols substitués (notamment des alkylphénols ou des arylphénols), des sels d'esters d'acides sulfosucciniques, des dérivés de la taurine (notamment des alkyltaurates), des esters phosphoriques d'alcools ou de phénols polyoxyéthylés, des esters d'acides gras et de polyols, les dérivés à fonction sulfates, sulfonates et phosphates des composés précédents. La présence d'au moins un agent tensioactif est généralement indispensable lorsque la matière active et/ou le support inerte ne sont pas solubles dans l'eau et que l'agent vecteur de l'application est l'eau.

D'une façon générale, les compositions selon l'invention contiennent habituellement de 0,05 à 95 % (en poids) de matière active, un ou plusieurs supports solides ou liquides et, éventuellement, un ou plusieurs agents tensioactifs. La teneur en agent tensioactif est avantageusement comprise entre 5 % et 40 % en poids. Sauf indication contraire les pourcentages donnés dans cette description sont des pourcentages pondéraux.

En fonction de la nature des supports, solides ou liquides, et des agents tensioactifs compris dans les compositions selon l'invention, celles-ci peuvent prendre des formes variées. Ces formes sont fonction du mode d'application souhaité de ladite composition.

Comme formes de compositions solides, on peut citer les poudres pour poudrage et les granulés, notamment ceux obtenus par extrusion, par compactage, par imprégnation d'un support granulé, par granulation à partir d'une poudre.

Comme formes de compositions liquides ou destinées à constituer des compositions liquides lors de l'application, on peut citer les solutions, en particulier les concentrés solubles dans l'eau, les émulsions, les suspensions concentrées, les poudres mouillables (ou poudre à pulvériser).

De manière plus générale, les compositions selon l'invention peuvent prendre de nombreuses formes de formulations. Ainsi, à titre d'exemple, on peut employer ces compositions sous forme de générateur d'aérosol, d'appât (prêt à l'emploi), de concentré pour préparation d'appâts, d'appât en stoc, d'appât sur grain, d'appât granulé, d'appât en plaquette, d'appât sur brisures, de suspension de capsules, de produit pour nébulisation a froid, de concentré émulsionnable, d'émulsion de type aqueux/aqueuse, d'émulsion de type huileux/inverse, de suspension concentrée pour traitement de semences, de gaz comprimé, de produit générateur de gaz, de produit pour nébulisation a chaud, de granulé, de granulé encapsulé, de granulé fin, de macrogranulé, de microgranulé, de granulés ou comprimés à disperser dans l'eau, de granulés ou comprimés solubles dans l'eau, de poudre pour poudrage, de poudre à disperser dans l'huile, de poudre pour traitement de semences à sec, de poudre soluble, de poudre de piste, de poudre mouillable pour traitement humide, de poudre soluble pour traitement de semences, ou de poudre mouillable, de suspension concentrée diluable dans l'huile, de liquide miscible dans l'huile, de pâte, de bâtonnet à usage agropharmaceutique, de semences traitées ou enrobées, de bougie fumigène, de cartouche fumigène, de fumigène, de granulé fumigène, de bâtonnet fumigène, de comprimé fumigène, de boite fumigène, de concentré soluble, de liquide pour traitement de semences, de suspension concentrée (= concentré fluidifiable), de liquide pour application à très bas volume, de suspension pour application à très bas volume, de produit diffuseur de vapeur.

Les compositions selon l'invention peuvent aussi contenir, en plus du composé pseudopeptidique de formule (I), une ou plusieurs autres matières actives, par exemple un ou plusieurs insecticides, fongicides, bactéricides, acaricides, arthropodicides, nématocides, attractants ou phéromones ou autres composés à activité biologique. Les compositions ainsi obtenues ont une activité à spectre élargi. Les compositions avec d'autres fongicides sont particulièrement avantageuses, par exemple les compositions avec les fongicides de la famille des triazoles, ou des dérivés de la strobilurine. Les compositions de la présente invention peuvent avantageusement contenir comme matière active supplémentaire l'azoxystrobine, le krésoxymméthyl, la discostrobine, le carbendazim, le thiram, le diéthofencarb, la dodine, le manèbe, le mancozèbe, le diflumetorim, l'ethirimol, le bénomyl, le cymoxanil, la fenpropidine, le fenpropimorph, le triadimefon, le captane, le captafol, le folpel, le thiophanate, le thiabendazole, l'acide phosphorique et ses dérivés comme le phosetyl-Al, le chlorothalonil, les compositions fongicides à base de cuivre, le dichloran, le metalaxyl, l'iprodione, la fénamidone, l'oxadixyl, la vinchlozoline, le tébuconazole, le bromuconazole, le triticonazole, le difenconazole, le diniconazole, le metconazole, le penconazole, le propiconazole, le prochloraz, le fénarimol, le triadiménol, le furalaxyl, les dérivés du cuivre comme l'hydroxyde et l'oxychlorure, le probénazole, l'époxyconazole, la famoxadone, la picoxystrobine, le fludioxonyl, le pyriméthanil, le mépanipyrim, le cyprodinyl, le quinoxyfen, la ferimzone, le fluazinam, la trifloxystrobine, le diméthomorphe, le bénalaxyl, la blasticidin-S, le fluquinconazole, le tricyclazole, le fluzilazole, les dérivés de la valinamide, comme par exemple l'iprovalicarb, le flutolanil, la guazatine, l'hexaconazole, l'hymexazol, l'isoprothiolane, la kazugamycine, le pencycuron, le phtalide, le pyroquilon, le tétraconazole, le thifluzamide et la carboxine.

La présente invention concerne également un procédé de lutte, à titre curatif ou préventif, contre les agents microbiens phytopathogènes des cultures. Ce procédé est caractérisé en ce que les semences, les feuilles ou les troncs de végétaux ou les surfaces, en particulier les sols, où poussent ou sont susceptibles de pousser ces végétaux sont traités par application, pulvérisation ou injection d'une quantité efficace et non phytotoxique d'un composé de formule (I) ou un de ses sels acceptables en agriculture ou un complexe métallique ou métalloïdique de ce composé également acceptable en agriculture, de préférence sous forme d'une composition antimicrobienne selon l'invention.

Par "quantité efficace et non phytotoxique", on entend une quantité de composé ou de composition selon l'invention suffisante pour permettre le contrôle ou la destruction des agents microbiens pathogènes (champignons, bactéries, virus) présents ou susceptibles d'apparaître sur les cultures, et n'entraînant pour lesdites cultures aucun symptôme notable de phytotoxicité. Une telle quantité est susceptible de varier dans de larges limites selon l'agent microbien à combattre, le type de culture, les conditions climatiques, et les composés compris dans la composition antimicrobienne selon l'invention. Cette quantité peut être déterminée par des essais systématiques au champ, à la portée de l'homme du métier.

Selon un mode particulier de réalisation de l'invention, les compositions selon l'invention sont utilisées pour traiter les semences, par exemple de céréales (blé, seigle, triticale et orge notamment), de pomme de terre, de coton, de pois, de colza, de maïs, de lin ou encore les semences d'arbres forestiers (notamment de résineux). On notera à ce propos que dans le jargon de l'homme de métier, le terme traitement de semences se rapporte au traitement des graines. Les techniques d'application sont bien connues de l'homme de métier et elles peuvent être utilisées sans inconvénient dans le cadre de la présente invention. On pourra citer par exemple le pelliculage ou l'enrobage.

L'invention concerne donc également un procédé de protection à titre préventif ou curatif des produits de multiplication des végétaux, ainsi que des végétaux en résultant, contre les maladies provoquées par des agents microbiens, caractérisé en ce que l'on recouvre lesdits produits d'une dose efficace et non phytotoxique d'une composition selon l'invention.

Parmi les produits de multiplications des végétaux concernés, on peut citer notamment les semences ou graines, et les tubercules.

Comme cela a été indiqué précédemment, les modalités de recouvrement des produits de multiplication des végétaux, notamment des semences, sont bien connues dans l'art et font appel en particulier aux techniques de pelliculage ou d'enrobage.

Selon un mode particulier de réalisation de l'invention, les compositions, ainsi que méthodes et procédés objets de la présente invention s'appliquent à des végétaux ou produits de multiplication desdits végétaux génétiquement transformés. Par génétiquement transformé, on entend des végétaux ou produits de multiplication des végétaux ayant intégré de manière stable dans leur génome un gène d'intérêt par les techniques de génie génétique. Parmi les gènes d'intérêt connus, on peut citer à titre d'exemple, des gènes codant une enzyme de résistance à un herbicide, par exemple le gène codant pour l'enzyme *bar* (White et al., NAR 18:1062, 1990) pour la tolérance au bialaphos, le gène codant pour l'enzyme EPSPS (US 5,188,642; WO 97/04103) pour la tolérance au glyphosate ou encore le gène codant pour l'enzyme HPPD (WO 96/38567) pour la tolérance aux isoxazoles. On peut également citer un gène codant pour une toxine insecticide, par exemple un gène codant pour une toxine de la bactérie *Bacillus thuringiensis* (pour exemple, voir International Patent Application WO 98/40490), pour la toxine de la bactérie *Xenorhabdus* (WO 85/00647, WO 98/08388, WO 98/50427, WO 99/03328, WO 99/54472 ou WO 00/30453) ou pour la toxine de la bactérie *Photorhabdus* (WO 97/17432 et WO 98/08932). On peut également citer des gènes de résistance aux maladies, par exemple un gène codant pour l'enzyme oxalate oxydase tel que décrit dans la demande de brevet EP 0 531 498 ou le brevet US 5,866,778, ou un gène codant pour un peptide antibactérien et/ou antifongique tels que ceux décrits dans les demandes de brevets WO 97/30082, WO 99/24594, WO 99/02717, WO 99/53053, et WO99/91089. On peut également citer des gène codant pour des caractères agronomiques de la plante, en particulier un gène codant pour une enzyme delta-6 désaturase tel que décrit dans les brevets US 5,552,306, US 5,614,313, et demandes de brevets WO 98/46763 et WO 98/46764, ou un gène codant pour une enzyme sérine acétyltransférase (SAT) tel que décrit dans les demandes de brevets WO 00/01833 et WO 00/36 127.

La présente invention concerne également des compositions vétérinaires ou pharmaceutiques comprenant comme matière active au moins une quantité efficace d'un composé pseudopeptidique de formule (I). De telles compositions permettent d'administrer le composé pseudopeptidique selon l'invention à des animaux ou des hommes de manière préventive ou curative, afin de les protéger contre des maladies d'origine microbienne. Une quantité efficace de composé correspond à une quantité de composé permettant de détruire ou d'inhiber la croissance des agents microbiens, et donc de stopper ou d'empêcher le développement d'une maladie d'origine microbienne.

Les compositions vétérinaires ou pharmaceutiques selon l'invention comprennent un composé de formule (I) et/ou un de ses sels physiologiquement acceptables et/ou un autre dérivé physiologiquement acceptable de ce composé, en association avec un support solide ou liquide, physiologiquement acceptable et/ou un agent diluant ou excipient également physiologiquement acceptable. D'une manière générale, ces compositions sont également appelées médicaments.

Par "physiologiquement acceptable", on entend selon l'invention que les sels, supports, agent diluant ou excipient ne soient pas nocifs pour les animaux ou les hommes destinés à recevoir la composition selon l'invention.

Les proportions et la nature des différents éléments entrant dans les compositions vétérinaires ou pharmaceutiques selon l'invention, en particulier les supports solides ou liquides et les agents diluants et excipients, sont déterminés en fonction du mode d'administration choisi et sont régis par les pratiques standards bien connues de l'homme du métier spécialisé dans la galénique des produits pharmaceutiques et vétérinaires.

Les compositions vétérinaires ou pharmaceutiques selon l'invention peuvent être administrées de manière parentérale, par exemple par une injection intramusculaire, sous-cutanée ou intra-péritonéale. Elles peuvent également être administrées par voie orale, en particulier par une absorption nasale ou buccale, ou par voie rectale. Lesdites compositions peuvent également être appliquées topiquement sur la peau afin de lutter contre les maladies cutanées causées par des agents microbiens.

Lorsque les compositions selon l'invention sont administrées de manière parentérale, celles-ci sont généralement liquides et comprennent un composé de formule (I) et au moins un agent diluant physiologiquement acceptable tel que par exemple, de l'eau, une solution saline, du dextrose ou d'autres diluants classiques connus de l'homme du métier. Les compositions selon l'invention peuvent également contenir un agent solubilisant, tel que par exemple le polyéthylène glycol, ou le polypropylène glycol. De telles compositions sont généralement stériles. La stérilisation peut se faire de plusieurs manières, par exemple par filtration ionisante, par incorporation d'agents stérilisants, par irradiation ou par chauffage.

Lorsque les compositions selon l'invention sont administrées par voie orale, elles peuvent prendre une forme solide ou liquide. Des compositions solides sont par exemple des comprimés, des pilules, des poudres ou des granulés. De telles compositions solides comprennent, en plus du composé de formule (I), un support inerte tel que par exemple l'amidon, la cellulose, le saccharose, le lactose ou la silice. Des compositions liquides sont par exemple des solutions, des suspensions, des émulsions des sirops ou des élixirs. De telles compositions liquides comprennent, en plus du composé de formule (I), un diluant inerte tel que l'eau, l'éthanol, le glycérol, des huiles végétales ou l'huile de paraffine. Ces compositions peuvent également prendre la forme de collyres, collutoires, gouttes nasales, ou aérosols.

Lorsque les compositions selon l'invention sont administrées par voie rectale, elles prennent généralement la forme d'un suppositoire ou d'une capsule rectale comprenant, en plus du composé de formule (I), au moins un excipient tel que par exemple le beurre de cacao, des glycérides semi-synthétiques ou des polyéthylène glycols.

Lorsque les compositions selon l'invention sont administrées de manière topique, elles prennent généralement la forme de crèmes ou de lotions.

L'invention concerne également l'utilisation du composé de formule (I) à titre de médicament pour le traitement et la prévention de maladies infectieuses, en particulier celles causées par des agents microbiens.

Les compositions vétérinaires ou pharmaceutiques selon l'invention peuvent également contenir, en plus du composé pseudopeptidique de formule (I), au moins une autre matière active représentée par un composé antimicrobien et/ou un composé a effet thérapeutique. Parmi les composés antimicrobiens, les compositions selon l'invention peuvent contenir par exemple des composés antibiotiques classiques comme ceux de la famille des pénicillines, ou des composés antimicrobiens de nature peptidique tels que ceux décrits dans les demandes de brevets WO 97/30082, WO 99/24594, WO 99/02717, WO 99/53053, et WO99/91089. Parmi les composés a effet thérapeutique, on entend tout composé dont l'activité permet de traiter une maladie humaine ou animale, d'origine infectieuse ou non.

### Description des figures

Figure 1: Chromatographe HPLC des composés pseudopeptidiques issus du milieu de culture des bactéries *Xenorhabdus nematophilus*. Cette étape finale de chromatographie (voir exemples 2 et 3) permet de séparer 15 fractions antimicrobiennes correspondant aux composés pseudopeptidiques selon l'invention.
Figures 2 et 3: Exemples de structures de composés pseudopeptidiques selon l'invention.

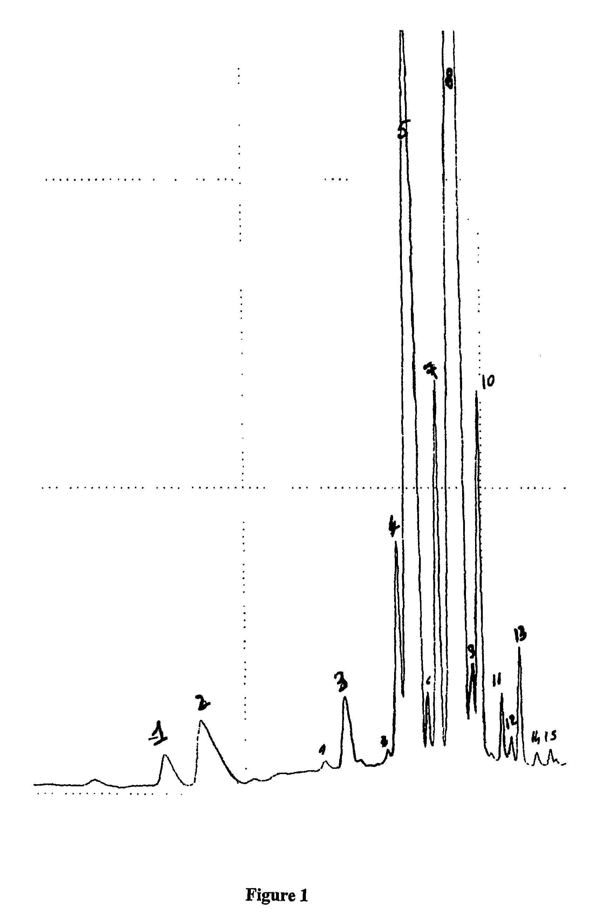

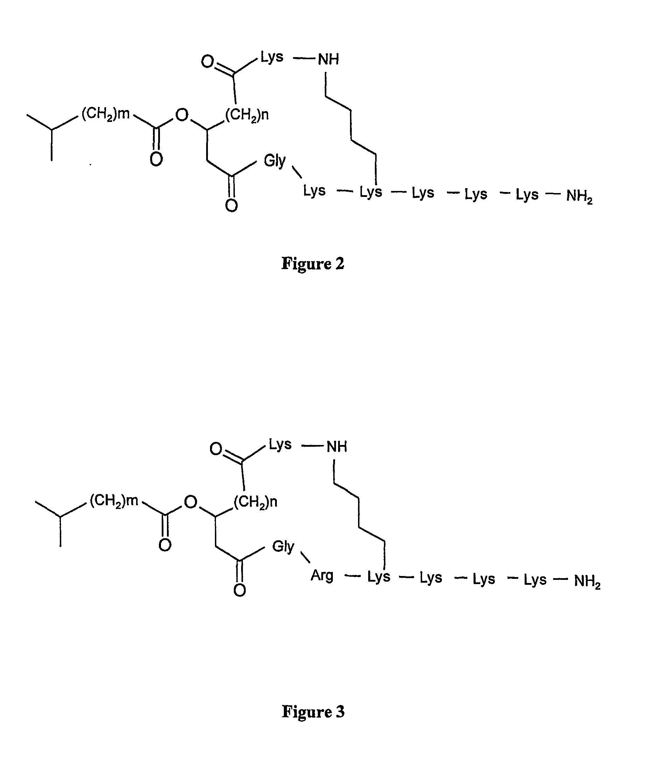

   Figure 2: Composé du pic 8 de la Figure 1
   Figure 3: Composé du pic 10 de la Figure 1

### Exemple 1: Souche, conditions de culture, et production des pseudopeptides

Les pseudopeptides selon l'invention peuvent être isolés à partir de la bactérie *Xenorhabdus nematophilus* (également nommée *X nematophila*; voir Euzeby and Boemare, 2000, Int. J. Syst. Evol. Microbiol. 50(4), 1691-1692), en particulier la souche *X. nematophilus* F1. La souche *X. nematophilus* F1 a été déposée dans la Collection Nationale de Culture de Microorganismes de l'Institut Pasteur (Paris, France) selon les dispositions du Traité de Budapest (1977).

De manière préférée, la souche *X. nematophilus* F1 se cultive en milieu non renouvelé dans des récipients de type Erlen. Par exemple, la culture peut se réaliser dans des Erlen d'un volume de 250 ml contenant 100 ml de milieu de culture. Les milieux de culture préférés sont le milieu LB (milieu de Luria-Bertani composé de 10g/l de Bactotryptone, de 5g/l d'extrait de levure, et de 5g/l de Chlorure de sodium) et le milieu TSB (milieu Trypticase Soja Broth composé de 30g/l de Trypcase soja et de 5g/l de Chlorure de sodium). La culture est réalisée dans un bain thermostaté à 28°C sous agitation et pour une durée de 48 heures. Les pseudopeptides sont généralement produits dans le milieu de culture après 30h d'incubation lorsque les cultures ont atteint la phase stationnaire de croissance bactérienne.

Les cultures peuvent également être mises en oeuvre sur milieux solides, en particulier sur des milieux gélosés tels que la Gélose Nutritive Ordinaire (GNO composée de 5g/l de bio-Gelytone, 3g/l d'extrait de viande de boeuf, 8g/l de NaCl et 15g/l d'Agar), le milieu LBA (composé de milieu LB auquel est ajouté de l'Agar purifié à 12 g/l), ou le milieu TSA (composé du milieu TSB auquel est ajouté de l'Agar purifié à 12 g/l).

### Exemple 2: Isolement des pseudopeptides

On peut isoler les pseudopeptides selon l'invention à partir du milieu de culture de la bactérie *Xenorhabdus nematophilus*, en particulier après centrifugation dudit milieu de culture à 6000 g pendant 15 min à 4°C. Le surnageant de centrifugation est récupéré, puis filtré sur un filtre dont le diamètre de pore est de 0,22 µm.

Le pH du surnageant est ensuite ajusté à pH 9 par ajout de soude concentrée. Le surnageant est alors passé sur une colonne de chromatographie de type cartouche SepPack Acell Plus CM (Waters) à raison de 100 ml de surnageant par cartouche. Le volume mort est éliminé. La cartouche est lavée avec 10 ml de tampon PBS (w/o Ca et Mg), puis éluée avec 10 ml de tampon NaCl 500 mM - Phosphate 20 mM, pH 9. Les 10 premiers millilitres d'élution sont récupérés, puis acidifiés avec 0,1 % (v/v) de TFA (Acide Tri-Fluoro acétique). Ils sont ensuite à nouveau passés sur une colonne de chromatographie de type cartouche SepPack Plus C18 (Waters). Le volume mort est éliminé. La cartouche est lavée avec 10 ml d'eau + 0,1 % (v/v) de TFA, puis éluée avec de l'acétonitrile à 100 % (v/v). Les 2 premiers ml d'élution sont récupérés, puis lyophilisés.

Le matériel lyophilisé est repris dans de l'eau acidifiée contenant 0,1 % (v/v) de TFA / Acétonitrile (75/25; v/v). Cet échantillon est passé sur une colonne de chromatographie de type colonne HPLC C18 RP8 *Symmetry shield* (Waters) a raison de 100 µg de matériel protéique par passage. La concentration protéique de l'échantillon est déterminée par la méthode BCA (Pierce). Le programme de chromatographie comprend une phase de lavage dans le mélange eau-TFA / acétonitrile (75/25 ; v/v), à raison de 1 ml/min pendant 10 min, puis une phase d'élution par gradient linéaire de 25 à 35% d'acétonitrile à raison de 1 ml/min, sur un intervalle de temps de 40 min. Les pseudopeptides sont récupérés dans des fractions de 0,5 ml puis lyophilisés. Si le matériel récupéré n'est pas pur après Spectrographie de masse par Electrospray, une deuxième HPLC selon le même protocole et avec la même colonne permet d'obtenir 100 % de pureté.

### Exemple 3: Caractérisation des pseudopeptides

La dernière étape de chromatographie (HPLC C18 RP8) a permis de séparer et d'identifier 15 fractions antimicrobiennes (pics de 1 à 15 sur la Figure 1). Les analyses du poids moléculaire au spectromètre de masse (Electrospray) des pics n° 5, 7, 8, 10, et 11 révèlent de petites molécules avec un PM compris entre 1052 et 1094 Da (voir tableau 1). Les échantillons ont été analysés dans un mélange H2O/Acétonitrile (50/50) + TFA à 0,01%, et le logiciel d'acquisition des données est MassLink (Micromasse).

**Tableau 1:**

| Pic n° | 5 | 7 | 8 | 10 | 11 |
|---|---|---|---|---|---|
| Masse Molaire (Da) | 1052 | 1080 | 1066 | 1094 | 1080 |

Les composés des pics 7 et 11 constituent des isomères de masse.

### Exemple 3.1: Analyse de la composition en acides aminés

Pour chaque analyse, une quantité de 100 µg de composé est nécessaire. Les échantillons lyophilisés sont repris dans 0,1 ml d'acide chlorhydrique à 5,7M, puis portés à 110°C pendant 24h pour une hydrolyse totale sous vapeurs d'acide. Après dérivation, les résidus peptidiques sont identifiés et dosés par chromatographie HPLC en phase reverse.

Les résultats obtenus ont permis d'identifier la nature peptidique des composés. Toutefois, l'analyse de la composition en acides aminés des composés correspondant aux pics n° 5, 7, 8, 10, et 11 a révélé deux familles de molécules hexapeptidiques de type:
Famille 1: Lys Lys Lys Lys Lys Gly (composés No. 5, 8 et 11)
Famille 2: Lys Lys Lys Lys Arg Gly (composés No. 7 et 10)

Cette analyse a également permis de montrer que ces composés antimicrobiens ne sont pas uniquement constitués d'acides aminés puisque la masse en acides aminés dosée par molécule est inférieure d'au moins 30% à la masse molaire des composés déterminée au spectromètre de masse (Tableau 1).

### Exemple 3.2: Séquençage N-terminal

Le séquençage N-terminal des composés 5 et 8 selon la méthode d'Edman a permis de montrer que 4 lysines sont reliées en position N-terminale, la position du résidu arginine n'étant pas déterminée. De plus, la différence entre la masse totale des composés et la masse de leur partie peptidique a conduit à la démonstration de la présence d'une structure supplémentaire indéterminée d'une masse molaire variant de 336 à 364 Da selon les molécules.

### Exemple 3.3: Analyse par Résonance Magnétique Nucléaire (RMN)

Les composés des pics 8 (représentant la famille 1) et 10 (représentant la famille 2) ont été isolés selon le protocole décrit à l'exemple 2 et leur masse contrôlée par Spectromètre de Masse-Electrospray avant analyse par RMN. Une quantité de 2 mg de chaque composé pur a été nécessaire.

L'analyse RMN a permis de préciser la structure pseudopeptidique des composés des deux familles. De plus, cette analyse a permis de déterminer que la partie peptidique de ces molécules est un heptapeptide, l'acide aminé basique n°5 étant reliée à la ramification aminée de l'acide aminé basique n°4. La structure des molécules 8 et 10 est donnée en Figures 2 et 3.

### Exemple 4: Efficacité des pseudopeptides vis-à-vis des champignons phytopathogènes

Les composés des pics 5, 7 et 8 de la figure 1 ont été testées vis-à-vis d'un ensemble de champignons phytopathogènes. Trois doses (10, 20, et 40 ppm) ont été employées pour chacun des composés.

L'activité antifongique a été détectée par un test d'inhibition de croissance en milieu liquide. Les spores des champignons à tester ont été mises en suspension dans un milieu de culture de type "Pomme de terre-Glucose". De préférence, on utilise 12 g de milieu Potato Dextrose Broth (Difco) pour 1 litre d'eau déminéralisée. Deux antibiotiques ont été rajoutés au milieu de culture: la tétracycline (concentration finale de 10 µg/ml) et la céfotaxime (100 µg/ml). On dépose 10 µl de chaque composé à tester dans des plaques de microtitration en présence de 90 µl de milieu de culture contenant les spores (à une concentration finale de 10⁴ spores/ml). L'incubation a été réalisée en chambre humide à 30°C durant 48 heures. La croissance fongique a été observée au microscope photonique après 24 h et quantifiée après 48 heures par mesure de l'absorbance à 600 nm à l'aide d'un spectrophotomètre lecteur de plaque de microtitration. L'activité des composés contre les champignons phytopathogènes est exprimée par un pourcentage d'inhibition de croissance correspondant à 100 x (1 - absorbance avec produit/ absorbance du témoin sans produit), l'absorbance étant mesurée à 600 nm, 5 jours après le début de l'expérience.

Les résultats de ce test sont présentés dans le tableau 3. Ils révèlent une excellente activité des trois composés, en particulier aux doses de 40 et 20 ppm. En outre, les trois composés présentent des spectres d'activité similaires vis-à-vis du groupe de champignons phytopathogènes testés. Les composés des pics 5 et 8 fournissent des résultats d'efficacité similaires, alors que le composé du pic 7 montre une efficacité légèrement plus faible sur le même spectre. Cette différence d'efficacité pourrait être liée à la composition en acides aminés des composés testés, identique pour les composés des pics 5 et 8 appartenant à la famille 1, et différente pour le composé du pic 7 appartenant à la famille 2.

**Tableau 3:**

| Activité des composés pseudopeptidiques issus des fractions 5, 7, et 8 vis-à-vis d'un ensemble de champignons phytopathogènes pour des doses de 10, 20, et 40 ppm. Les résultats sont exprimés en pourcentage d'inhibition de croissance des champignons testés. | | | | | | | | | |
|---|---|---|---|---|---|---|---|---|---|
| | **Composé 5** | | | **Composé 7** | | | **Composé 8** | | |
| **Dose appliquée** | **10 ppm** | **20 ppm** | **40 ppm** | **10 ppm** | **20 ppm** | **40 ppm** | **10 ppm** | **20 ppm** | **40ppm** |
| *Alternaria brassicae* | 82 | 84 | 83 | 25 | 47 | 83 | 82 | 82 | 83 |
| *Botrytis cinerea sp* | 38 | 95 | 95 | 53 | 12 | 83 | 52 | 95 | 94 |
| *Botrytis cinerea myc* | 21 | 34 | 60 | 0 | 6 | 34 | 40 | 70 | 93 |
| *Cladosporium sp* | 94 | 94 | 94 | 74 | 95 | 95 | 94 | 94 | 94 |
| *Cladosporium myc* | 89 | 90 | 89 | 0 | 40 | 91 | 70 | 92 | 91 |
| *Fusarium culmorum sp* | 96 | 96 | 96 | 66 | 97 | 97 | 96 | 96 | 96 |
| *Helminthosporium teres sp* | 70 | 91 | 95 | 33 | 47 | 80 | 76 | 94 | 92 |
| *Rhizoctonia solani myc* | 9.7 | 85 | 93 | 6 | 7 | 64 | 56 | 91 | 93 |
| *Phytophthora myc* | 79 | 78 | 77 | 71 | 81 | 78 | 76 | 78 | 77 |
| *Piricularia oryzae sp* | 18 | 22 | 45 | 12 | 15 | 21 | 30 | 35 | 45 |
| *Septoria tritici sp* | 88 | 88 | 85 | 0 | 86 | 89 | 87 | 88 | 86 |

### Exemple 5: Efficacité des pseudopeptides vis-à-vis des bactéries

Deux tests ont été utilisés pour mesurer l'activité antibactérienne des pseudopeptides, un test d'inhibition de la croissance bactérienne en microplaques (de 96 puits) en milieu liquide et un test d'inhibition de la croissance bactérienne en boîte de Pétri en milieu solide.
* ***Test en microplaques:*** La culture bactérienne à tester est prélevée en phase exponentielle, puis diluée au 1/500^{ème} dans du milieu liquide TSB frais. Chaque puits de la microplaque est rempli avec 100 µl de suspension bactérienne diluée. Dans le premier puits, 100 µl de l'échantillon de pseudopeptide à tester est ajouté à la suspension bactérienne, puis l'échantillon est dilué de 2 en 2 en transférant d'un puits à l'autre 100 µl de suspension. La lecture de la microplaque se fait en mesurant la densité optique à 660 nm (DO₆₀₀ₙₘ) après 24h d'incubation à 28°C ou 37°C selon les bactéries cibles. Connaissant la concentration en pseudopeptide de l'échantillon de départ, ce test permet de déterminer la valeur de la CMI (Concentration Minimale d'Inhibition de la croissance bactérienne). Dans ce test, la CMI correspond à la concentration de l'échantillon pour lequel la DO₆₀₀ₙₘ est inférieure à celle du témoin de culture.
* ***Test en boîte de Pétri****:* La culture bactérienne à tester est prélevée en phase exponentielle, puis incorporée à 10 ml d'une gélose dite "sous-couche" (Agarose 1% poids/vol ; NaCl 100 mM ; Trypcase 0.03 % poids/vol ; Tampon phosphate 10 mM pH7.4) à raison de 2.10⁵ bactéries/ml. Le mélange est coulé dans une boîte de Pétri ordinaire. 9 puits d'environ 10 µl de volume sont formés dans la "sous-couche" à l'aide d'un embout de pipette stérile de 10 ml. Ces puits sont remplis avec une série de dilutions des pseudopeptides à tester (concentration initiale 500 µg/ml, et dilution de deux en deux jusqu'à 1,95 µg/ml). Après diffusion à température ambiante pendant 2h, une gélose dite "sur-couche" (Agarose 1% wt/vol ; Trypcase 6 % wt/vol ; Tampon phosphate 10 mM pH7.4) est ajoutée sur la sous-couche à raison de 5 à 10 ml. Après solidification, l'ensemble est mis à incuber pendant une nuit. Le diamètre d'inhibition de la culture bactérienne autour de chaque puits est mesuré en mm; il existe une relation linéaire entre le diamètre d'inhibition et le log₁₀ de la concentration en pseudopeptide qui permet de déterminer par extrapolation la valeur de la CMI.

Une première série de résultats a été obtenue en utilisant le test en boîte de Pétri vis-à-vis de bactéries non pathogènes issues de la collection de Travaux pratiques du laboratoire de Pathologie Comparée de l'Université de Montpellier (France). Dans cette série, les pseudopeptides des pics 5, 7, 8,10 et 11 ont été testés (Tableau 4).

**Tableau 4:**

| Valeurs des CMI en µg/ml de différentes fractions contenant des pseudopeptides vis-à-vis de plusieurs espèces de bactéries. La polymixine est utilisée à titre de contrôle positif d'inhibition. | | | | | | |
|---|---|---|---|---|---|---|
| | **Composé 5** | **Composé 7** | **Composé 8** | **Composé 10** | **Composé 11** | **Polymixine** |
| *Escherichia coli CIP548* | 15 | 15 | 7,5 | 7,5 | 15 | 0,25 |
| *Salmonella sp.* | 30 | 15 | 30 | 30 | 15 | 0,5 |
| *Klebsiella pneumoniae* | 30 | 30 | 15 | 15 | 30 | - |
| *Proteus vulgaris CIP58.60* | 200 | 100 | 100 | 200 | 200 | 50 |
| *Serratia entomophila* | 60 | 60 | 30 | 15 | 30 | - |
| *Staphylococcus aureus* | 7,5 | 15 | 15 | 7,5 | 7,5 | 20 |
| *Staphylococcus epidermidis* | 30 | - | 30 | - | - | 20 |
| *Streptococcus Gr. D* | 200 | - | 200 | - | - | - |
| *Pseudomonas fluorescens* | 3 | 1,5 | 3 | 1,5 | 1,5 | - |
| *Bacillus megaterium* | 0,75 | 1,5 | 1,5 | 0,75 | 0,75 | - |

Une seconde série de résultats a été obtenue en utilisant le test en microplaques vis-à-vis de bactéries nosocomiales issues de la collection du laboratoire de Bactériologie Médicale du Centre Hospitalier Universitaire de Nîmes (France). Dans cette série, seul le pseudopeptide contenu dans la fraction 8 a été testé (Tableau 5).

**Tableau 5 :**

| Valeurs des CMI en µg/ml du pseudopeptide contenu dans la fraction 8 vis-à-vis de différentes bactéries nosocomiales. | | | | | |
|---|---|---|---|---|---|
| | *Enterococcus sp.* | *Enterobacter aerogenes* | *Pseudomonas aeruginosa* | *Staphylococcus aureus* | *Stenotrophomonas maltophilia* |
| Fraction 8 | 12,5 | 25 | 12,5 | 25 | 25 |

Le tableau 4 montre que l'activité antibactérienne varie peu entre les pseudopeptides. Le spectre d'activité antibactérien des pseudopeptides est large puisqu'il comprend des bactéries appartenant à des espèces éloignées sur le plan phylogénétique. En outre, ce spectre comprend des bactéries à Gram positif et des bactéries à Gram négatif. La CMI des pseudopeptides selon l'invention est relativement élevée, indiquant une activité spécifique assez limitée. Toutefois, quelques bactéries sont sensibles à faible dose (ex : *P. fluorescens*).

De plus, les pseudopeptides selon l'invention présentent une activité antibactérienne vis-à-vis de 5 souches de bactéries nosocomiales résistantes aux antibiotiques médicaux actuels. Ce résultat démontre le potentiel pharmaceutique de ces molécules.

### Exemple 6: Efficacité des pseudopeptides vis-à-vis des virus

L'activité antivirale des pseudopeptides selon l'invention a été démontrée vis-à-vis du bactériophage lambda. Le bactériophage λ-ZAP est naturellement lytique sur la souche *E. coli* SURE (Stratagène). Cette activité lytique est mesurable par la méthode des plages de lyse en gélose Top-Agar. 2 µl d'une suspension de virus à 3,5 .10⁵ pfu/ml dans du tampon SM ont été mis en présence de 2 µl du pseudopeptides compris dans le pic 8 à 0,5 mg/ml dans de l'eau pure. Après 15 min d'incubation à 37°C, la suspension est mélangée à 3,5 ml de Top-Agar pré-ensemencée avec *E. coli* SURE à 2 .10⁵ cfu/ml. La concentration de pseudopeptide dans la gélose Top-Agar est inférieure à la CMI vis-à-vis de *E. coli* SURE. Le mélange est coulé en boîte de Pétri puis mis à incuber à 37°C pendant 12h. Le résultat est donné dans le tableau 6.

**Tableau 6:**

| Effet du pseudopeptide compris dans le pic 8 sur le nombre de pfu de bactériophage lambda par boite de culture. | | |
|---|---|---|
| | En présence de pseudopeptide | En absence de pseudopeptide |
| Nbre de pfu/boîte | 40 ± 5 | 700 ±50 |

L'inhibition du cycle lytique du bactériophage lambda est drastique (>95%) en présence du pseudopeptides compris dans le pic 8 à 250 µg/ml. Cette inhibition peut être extrapolée aux pseudopeptides compris dans les autres pics de la Figure 1. De plus, le bactériophage lambda étant un virus encapsidé, cette inhibition pourrait donc concerner, au minimum, tous les virus dits "enveloppés".

### Exemple 7: Propriétés physico-chimiques des pseudopeptides

Les effets de traitements physico-chimiques sur la stabilité de l'activité des pseudopeptides ont été mesurés. Afin de déterminer les effets de tels traitements, les pseudopeptides ont été soumis à un traitement particulier, replacés dans des conditions standards (dans un tampon PBS), puis utilisés dans le test en boîtes de Pétri pour mesurer leur activité antimicrobienne après le traitement vis-à-vis des bactéries *Micrococcus luteus* et *Escherichia coli.*
* ***Traitements thermiques:*** Un volume de 0,5 ml de solution de pseudopeptide à 500 µg/ml dans du PBS est porté pendant 60 min à 37, 60, 10, et 120°C. Après refroidissement, l'activité de chaque pseudopeptide est mesurée. Aucun traitement de température n'a eu d'effet sur les valeurs de CMI vis-à-vis de *Micrococcus luteus* et *Escherichia coli* par comparaison aux contrôles.
* ***Traitements enzymatiques:*** Un volume de 0,5 ml de solution de pseudopeptide à 500 µg/ml dans le tampon d'enzyme est incubé pendant 60 min à 37°C en présence de 5 à 10 unités enzymatiques par ml. Après incubation, l'enzyme est précipité par ajout d'acide trichloracétique à 0,5M de concentration finale, le surnageant est lyophilisé puis repris dans 0,5 ml de tampon PBS pour mesure de l'activité. Les enzymes testées sont: Thermolysine (Sigma, Protéase X, réf. P1512); Trypsine (Sigma, type I, réf. T8003); Subtilisine de Carlsberg (Sigma, Protéase VIII, réf. P5380); Pronase E (Sigma, Protéase XIV, réf. P5147); Endoprotéinase Lys-C (Sigma, réf. P2289); Lipase (Sigma, type XIII, réf. L9518); Phospholipase A2 (Sigma, réf. P9279); Phospholipase C (Sigma, type IX, réf. P1392). Aucun traitement enzymatique, à l'exception de la Pronase E, n'a eu d'effet sur les valeurs de CMI vis-à-vis de *Micrococcus luteus* et *Escherichia coli* par comparaison aux contrôles. La Pronase E supprime totalement l'activité antibactérienne des pseudopeptides.
* ***Traitements pH:*** Un volume de 0,5 ml de solution de pseudopeptide à 500 µg/ml dans un tampon au pH désiré est incubé pendant 24h à température ambiante. Après incubation, la solution est lyophilisée et le précipité de pseudopeptides est repris dans 0,5 ml de tampon PBS pour mesure de l'activité. Les conditions de pH testées ont été pH 4, pH 6, pH 8 et pH 10. Aucun traitement de pH n'a eu d'effet sur les valeurs de CMI vis-à-vis de *Micrococcus luteus* et *Escherichia coli* par comparaison aux contrôles.

Ces résultats démontrent que les pseudopeptides sont des molécules très stables. Elles résistent en particulier aux pH acides et aux températures élevées.

### Exemple 8: Bactéries sources de pseudopeptides

Les pseudopeptides ont été recherchés dans d'autres souches bactériennes du genre *Xenorhabdus*, ainsi que dans des souches du genre *Photorhabdus*. Les différentes souches bactériennes sont issues de la collection du laboratoire de Pathologie Comparée de l'Université de Montpellier (France).
Chaque souche a été mise en culture selon les conditions de culture utilisée pour *X. nematophilus* F1 telles que décrites dans l'exemple 1. De plus, les surnageants de culture ont subi la première partie du protocole d'extraction décrit à l'exemple 2, à savoir le passage sur les cartouches de chromatographie SepPack CM et SepPack C18. Ensuite, l'activité antibactérienne de la fraction d'élution issue de la cartouche SepPack C18 a été mesurée qualitativement pour chaque souche en utilisant le test d'inhibition de croissance bactérienne en milieu solide vis-à-vis de plusieurs souches bactériennes et d'une souche de levure du genre Candida (Tableau 7).

**Tableau 7:**

| Activité antibactérienne des extraits de cultures de bactéries des genres *Xenorhabdus* et *Photorhabdus* vis-à-vis de plusieurs souches bactériennes et d'une souche de levure du genre Candida. (Str = *Streptococcus* Groupe D; Sta = *Staphylococcus aureus*; Mic *= Micrococcus luteus*; Bac = *Bacillus megaterium*; Pse = *Pseudomonas fluorescens*; Esc = *Escherichia coli*; Ser *= Serratia marcescens*; Can = *Candida* sp.). Les souches dont le nom est suivi de /1 sont les souches sauvages telles qu'isolées des nématodes, et celles dont le nom est suivi de /2 sont des variants spontanés des souches sauvages. | | | | | | | | | | |
|---|---|---|---|---|---|---|---|---|---|---|
| **Genre** | **Espèce** | **Souche** | **Str** | **Sta** | **Mic** | **Bac** | **Pse** | **Esc** | **Ser** | **Can** |
| *Xenorhabdus* | *nematophila* | F1/1 | + | + | + | + | + | + | w | + |
| *Xenorhabdus* | *nematophila* | F1/2 | - | - | - | - | - | - | - | - |
| *Xenorhabdus* | *nematophila* | AN6/1 | - | + | + | + | - | - | - | - |
| *Xenorhabdus* | *japonica* | JPO2/1 | + | + | + | + | + | + | w | + |
| *Xenorhabdus* | *beddingii* | Q58/1 | + | + | + | + | w | - | - | + |
| *Xenorhabdus* | *beddingii* | Q58/2 | - | - | - | - | - | - | - | - |
| *Xenorhabdus* | *poinarii* | G6/1 | - | - | - | - | - | - | - | - |
| *Xenorhabdus* | *poinarii* | G6/2 | - | - | - | - | - | - | - | - |
| *Xenorhabdus* | *sp*. | Sav/1 | - | + | + | + | + | w | - | + |
| *Xenorhabdus* | *sp*. | Sav/2 | - | - | - | - | - | - | - | - |
| *Xenorhabdus* | *sp*. | K77/1 | + | + | + | + | + | + | w | + |
| *Xenorhabdus* | *sp*. | K77/2 | - | - | - | - | - | - | - | - |
| *Xenorhabdus* | *bovienii* | F3/1 | + | + | w | + | + | + | - | w |
| *Xenorhabdus* | *bovienii* | F3/2 | - | - | - | - | - | - | - | - |
| *Xenorhabdus* | *bovienii* | Si/1 | + | + | + | w | + | + | + | + |
| *Xenorhabdus* | *bovienii* | Si/2 | - | - | - | - | - | - | - | - |
| *Xenorhabdus* | *bovienii* | T228/1 | + | + | + | + | + | + | + | + |
| *Xenorhabdus* | *bovienii* | T228/2 | - | - | - | - | - | - | - | - |
| *Xenorhabdus* | *sp*. | VC01/1 | w | + | + | + | w | w | - | + |
| *Xenorhabdus* | *sp*. | FRM16/1 | - | + | + | + | + | w | - | + |
| *Xenorhabdus* | *sp*. | KR1/1 | + | + | + | + | + | + | + | + |
| *Xenorhabdus* | *sp*. | KR2/2 | - | - | - | - | - | - | - | - |
| *Xenorhabdus* | *sp*. | UY61/1 | + | + | + | + | + | + | + | + |
| *Xenorhabdus* | *sp*. | SE1/1 | + | + | + | + | + | + | + | + |
| *Xenorhabdus* | *sp*. | SE1/2 | - | - | - | - | - | - | - | - |
| *Xenorhabdus* | *sp*. | JM26/1 | + | + | + | + | + | + | + | + |
| *Photorhabdus* | *asymbiotica* | 1216-79/1 | - | - | - | - | - | - | - | - |
| *Photorhabdus* | *asymbiotica* | 1216-79/2 | - | - | - | - | - | - | - | - |
| *Photorhabdus* | *luminescens* | FRG26/1 | - | - | - | - | - | - | - | - |
| *Photorhabdus* | *sp*. | Q614/1 | - | - | - | - | - | - | - | - |
| *Photorhabdus* | *sp*. | Q614/2 | - | - | - | - | - | - | - | - |
| *Photorhabdus* | *luminescens* | Hb/1 | - | - | - | - | - | - | - | - |
| *Photorhabdus* | *luminescens* | HB/2 | - | - | - | - | - | - | - | - |
| *Photorhabdus* | *luminescens* | C8406/1 | - | - | - | - | - | - | - | - |
| *Photorhabdus* | *luminescens* | C84/06/2 | - | - | - | - | - | - | - | - |
| *Photorhabdus* | *Luminescens akhurstii* | JM12/1 | - | - | - | - | - | - | - | - |
| *Photorhabdus* | *luminescens akhurstii* | FRG04/1 | - | - | - | - | - | - | - | - |
| *Photorhabdus* | *luminescens laimondii* | TTO1/1 | - | - | - | - | - | - | - | - |
| *Photorhabdus* | *temperata* | XLNach/1 | *-* | - | - | - | - | - | - | - |
| *Photorhabdus* | *temperata* | XLNach/2 | *-* | - | - | - | - | - | - | - |
| *Photorhabdus* | *temperata* | Meg/1 | - | - | - | - | - | - | - | - |
| *Photorhabdus* | *temperata* | Meg/2 | - | - | - | - | - | - | - | - |
| *Photorhabdus* | *temperata* | C1/1 | - | - | - | - | - | - | - | - |
| *Photorhabdus* | *temperata* | C1/2 | - | - | - | - | - | - | - | - |
| *Photorhabdus* | *temperata* | NZH3/1 | - | - | - | - | - | - | - | - |
| *Photorhabdus* | *temperata* | NZH3/2 | - | - | - | - | - | - | - | - |

Toutes les souches de *Xenorhabdus*, à l'exception de la souche *X. poinarii*, produisent des pseudopeptides à activité antibactérienne. En revanche, aucune des souches de *Photorhabdus* n'en produit. En outre, tous les pseudopeptides produits par les souches de *Xenorhabdus* ont, comme ceux de la souche *X. nematophilus* F1, un spectre d'activité large vis-à-vis des bactéries à Gram positif et des bactéries à Gram négatif, ainsi que vis-à-vis d'une levure. Enfin, aucun des variants de phase 2 (souches /2) ne produit de pseudopeptides.

## Revendications

1. Composé pseudopeptidique antimicrobien, **caractérisé en ce qu'**il est de formule (I) 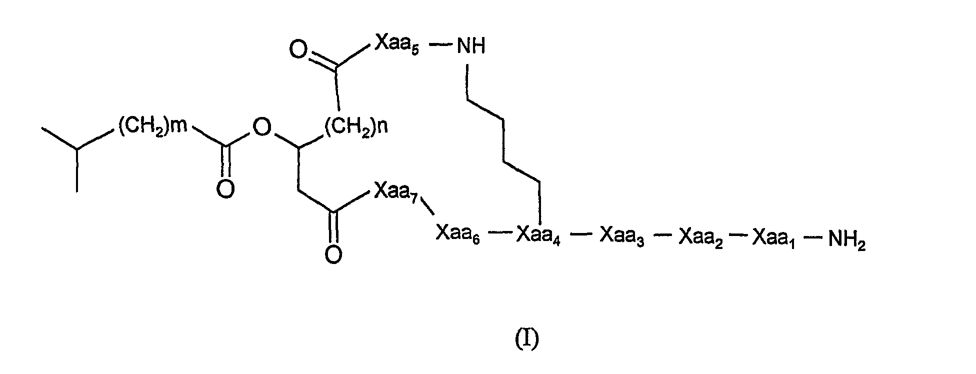 dans laquelle:
- Xaa (1 à 6) est un résidu acide aminé basique, de préférence la Lysine ou l'Arginine
- Xaa 7 est la glycine ou un résidu acide aminé à chaîne latérale aliphatique
- m = 1 ou 2 et n = 2 ou 3

2. Composé pseudopeptidique antimicrobien selon la revendication 1, **caractérisé en ce que** les résidus Xaa 1 à 6 sont des résidus Lysine.

3. Composé pseudopeptidique antimicrobien selon la revendication 1, **caractérisé en ce que** les résidus Xaa 1, 2, 3, 4, et 5 sont des résidus Lysine, et le résidu Xaa 6 est un résidu Arginine.

4. Composé pseudopeptidique antimicrobien selon l'une des revendications 1 à 3, **caractérisé en ce que** le résidu Xaa 7 est un résidu Glycine

5. Composé pseudopeptidique antimicrobien selon l'une des revendications 1 à 4, **caractérisé en ce que** m est égal à 1 et n est égal à 3

6. Composé pseudopeptidique antimicrobien selon l'une des revendications 1 à 4, **caractérisé en ce que** m est égal à 2 et n est égal à 2

7. Composé pseudopeptidique antimicrobien selon l'une des revendications 1 à 6, **caractérisé en ce qu'**il est isolé à partir d'une bactérie du genre *Xenorhabdus*

8. Composé pseudopeptidique antimicrobien selon la revendication 7, **caractérisé en ce qu'**il est isolé à partir de la bactérie *Xenorhabdus nematophilus*

9. Composé pseudopeptidique antimicrobien selon l'une des revendications 1 à 8, **caractérisé en ce qu'**il est antifongique

10. Composé pseudopeptidique antimicrobien selon la revendication 9, **caractérisé en ce qu'**il est actif vis-à-vis des champignons phytopathogènes

11. Composé pseudopeptidique antimicrobien selon la revendication 10, **caractérisé en ce qu'**il est actif contre les champignons du genre *Botrytis*, en particulier *B. cinerea*, du genre *Piricularia,* en particulier *P. oryzae*, du genre *Helminthosporium*, en particulier *H. teres*, du genre *Fusarium,* en particulier *F*. *culmorum*, du genre *Septoria*, en particulier *S. tritici*, du genre *Alternaria,* en particulier *A. brassicae*, du genre *Rhizoctonia*, en particulier *R. solani*, du genre *Phytophtora*, et du genre *Cladosporium*.

12. Composé pseudopeptidique antimicrobien selon la revendication 9, **caractérisé en ce qu'**il est actif vis-à-vis des champignons pathogènes de l'homme

13. Composé pseudopeptidique antimicrobien selon la revendication 12, **caractérisé en ce qu'**il est actif contre les champignons du genre *Candida*

14. Composé pseudopeptidique antimicrobien selon l'une des revendications 1 à 8, **caractérisé en ce qu'**il est antibactérien

15. Composé pseudopeptidique antimicrobien selon la revendication 14, **caractérisé en ce qu'**il est actif vis-à-vis des bactéries du genre *Escherichia*, en particulier *E. coli*, du genre *Salmonella*, du genre *Klebsiella*, en particulier du *K*. *pneumoniae*, du genre *Proteus,* en particulier, *P. vulgaris*, du genre *Serratia*, en particulier *S. entomophila*, du genre *Staphylococcus*, en particulier *S. epidermis*, du genre *Steptococcus,* du genre *Pseudomonas*, en particulier *P. fluorescens*, du genre *Bacillus*, en particulier *B. megaterium*.

16. Composé pseudopeptidique antimicrobien selon la revendication 14, **caractérisé en ce qu'**il est actif vis-à-vis des bactéries nosocomiales

17. Composé pseudopeptidique antimicrobien selon la revendication 16, **caractérisé en ce qu'**il est actif vis-à-vis des bactéries du genre *Staphylococcus,* en particulier *S*. *aureus*, du genre *Pseudomonas*, en particulier *P. aeruginosa*, du genre *Enterococcus,* du genre *Enterobacter*, en particulier *E. aerogenes*, ou du genre *Stenotrophomonas,* en particulier *S*. *maltophila*.

18. Composé pseudopeptidique antimicrobien selon l'une des revendications 1 à 8, **caractérisé en ce qu'**il est antiviral

19. Composé pseudopeptidique antimicrobien selon la revendication 18, **caractérisé en ce qu'**il est actif vis-à-vis des virus encapsidés

20. Composé pseudopeptidique antimicrobien selon la revendication 19, **caractérisé en ce qu'**il est actif vis-à-vis des phages

21. Composé pseudopeptidique antimicrobien selon la revendication 20, **caractérisé en ce qu'**il est actif vis-à-vis du phage lambda

22. Procédé de production d'un composé pseudopeptidique antimicrobien selon l'une des revendications 1 à 21, **caractérisé en ce qu'**il comprend les étapes de:
(a) mise en culture d'une bactérie du genre *Xenorhabdus* jusqu'à atteinte de la phase stationnaire de culture
(b) isolement du surnageant de culture
(c) séparation des composés contenus dans le surnageant par passage dudit surnageant dans au moins une colonne de chromatographie
(d) isolement sélectif d'au moins un composé selon l'une des revendications 1 à 21 par isolement des fractions d'élution correspondant aux pics de chromatographie

23. Procédé selon la revendication 22, **caractérisé en ce que** la bactérie du genre *Xenorhabdus* est une bactérie de l'espèce *Xenorhabdus nematophilus*

24. Procédé selon l'une des revendications 22 ou 23, **caractérisé en ce que** l'isolement du surnageant de culture se fait par centrifugation de ladite culture

25. Procédé selon l'une des revendications 22 ou 23, **caractérisé en ce que** l'isolement du surnageant de culture se fait par filtration de ladite culture

26. Composition agrochimique, **caractérisée en ce qu'**elle comprend une quantité efficace d'un composé selon l'une des revendications 1 à 21 ou un de ses sels acceptables en agriculture ou un complexe métallique ou métalloïdique de ce composé également acceptable en agriculture

27. Composition agrochimique selon la revendication 26, **caractérisée en ce qu'**elle comprend en plus au moins un support inerte et/ou au moins un agent tensio-actif

28. Composition agrochimique selon l'une des revendications 26 ou 27, **caractérisée en ce qu'**elle est utilisée pour les traitements préventifs ou curatifs contre les maladies des plantes causées par des agents microbiens phytopathogènes

29. Composition agrochimique selon la revendication 28, **caractérisée en** ce les maladies sont causées par des champignons phytopathogènes

30. Composition agrochimique selon la revendication 28, **caractérisée en ce** les maladies sont causées par des bactéries phytopathogènes

31. Composition agrochimique selon la revendication 28, **caractérisée en ce** les maladies sont causées par des virus phytopathogènes

32. Composition agrochimique selon l'une des revendications 26 à 31, **caractérisée en ce qu'**elle contient, en plus du composé pseudopeptidique de formule (I), au moins une autre matière active

33. Composition agrochimique selon la revendication 32, **caractérisée en ce que** la matière active supplémentaire-est un composé fongicide

34. Procédé de lutte à titre curatif ou préventif contre les agents microbiens phytopathogènes des cultures, **caractérisé en ce qu'**une quantité efficace d'une composition selon l'une des revendications 26 à 33 est appliquée sur les semences, les feuilles ou les troncs de végétaux, ou les surfaces, en particulier les sols, où poussent ou sont susceptibles de pousser ces végétaux

35. Procédé de protection à titre préventif ou curatif des produits de multiplication des végétaux, ainsi que des végétaux en résultant, contre les maladies provoquées par des agents microbiens, **caractérisée en ce que** l'on recouvre lesdits produits d'une dose efficace et non phytotoxique d'une composition selon l'une des revendications 26 à 33

36. Procédé selon l'une des revendications 34 ou 35, **caractérisé en ce que** les végétaux sont des végétaux cultivés

37. Procédé selon la revendication 36, **caractérisé en ce que** les végétaux cultivés sont des végétaux génétiquement transformés

38. Procédé selon l'une des revendications 36 ou 37, **caractérisé en ce que** les végétaux cultivés sont des céréales, des légumineuses, des arbres fruitiers, des arbres forestiers, de la vigne, des cultures oléagineuses, des cultures maraîchères, des solanées ou de la betterave.

39. Procédé selon la revendication 38, **caractérisé en ce que** les céréales sont le Blé, le Mais, le Riz, l'Orge, l'Avoine

40. Composition pharmaceutique ou vétérinaire, **caractérisée en ce qu'**elle comprend au moins une quantité efficace d'un composé selon l'une des revendications 1 à 21 ou un de ses sels physiologiquement acceptables ou un complexe métallique ou métalloïdique de ce composé également physiologiquement acceptable

41. Composition pharmaceutique ou vétérinaire selon la revendication 40, **caractérisée en ce qu'**elle comprend en plus au moins un support inerte et/ou au moins un agent diluant ou excipient

42. Composition pharmaceutique ou vétérinaire selon l'une des revendications 40 ou 41, **caractérisée en ce qu'**elle est utilisée pour les traitements préventifs ou curatifs contre des maladies humaines ou animales causées par des agents microbiens pathogènes

43. Composition pharmaceutique ou vétérinaire selon la revendication 42, **caractérisée en ce qu'**elle comprend, en plus du composé pseudopeptidique de formule (I), au moins une autre matière active représentée par un composé antimicrobien et/ou un composé a effet thérapeutique

44. Composé selon l'une des revendications 1 à 21 pour utilisation à titre de médicament dans le traitement ou la prévention des maladies infectieuses

## Patentansprüche

1. Pseudopeptidische antimikrobielle Verbindung, **dadurch gekennzeichnet, dass** sie die Formel (I) 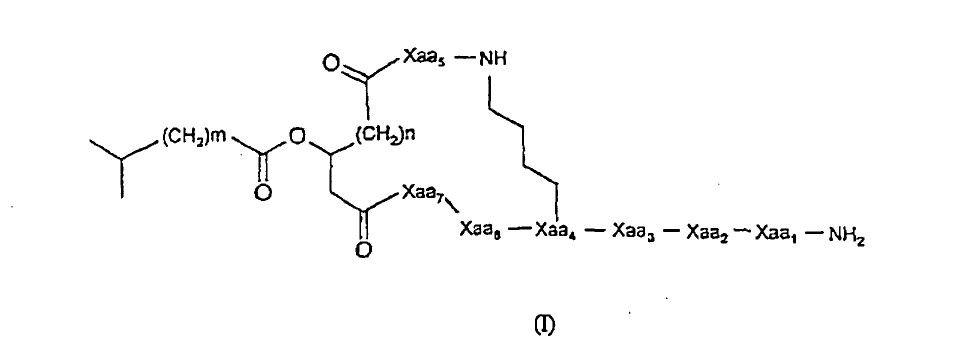 aufweist, in welcher:
- Xaa (1 bis 6) ein basischer Aminosäurerest, vorzugsweise Lysin oder Arginin ist,
- Xaa 7 Glycin oder ein Aminosäurerest mit aliphatischer Seitenkette ist,
- m = 1 oder 2 und n = 2 oder 3.

2. Pseudopeptidische antimikrobielle Verbindung nach Anspruch 1, **dadurch gekennzeichnet, dass** die Reste Xaa 1 bis 6 Lysinreste sind.

3. Pseudopeptidische antimikrobielle Verbindung nach Anspruch 1, **dadurch gekennzeichnet, dass** die Reste Xaa 1, 2, 3, 4 und 5 Lysinreste sind und der Rest Xaa 6 ein Argininrest ist.

4. Pseudopeptidische antimikrobielle Verbindung nach einem der Ansprüche 1 bis 3, **dadurch gekennzeichnet, dass** der Rest Xaa 7 ein Glycinrest ist.

5. Pseudopeptidische antimikrobielle Verbindung nach einem der Ansprüche 1 bis 4, **dadurch gekennzeichnet, dass** m gleich 1 ist und n gleich 3 ist.

6. Pseudopeptidische antimikrobielle Verbindung nach einem der Ansprüche 1 bis 4, **dadurch gekennzeichnet, dass** m gleich 2 ist und n gleich 2 ist.

7. Pseudopeptidische antimikrobielle Verbindung nach einem der Ansprüche 1 bis 6, **dadurch gekennzeichnet, dass** sie ausgehend von einem Bakterium der Gattung *Xenorhabdus* isoliert wird.

8. Pseudopeptidische antimikrobielle Verbindung nach Anspruch 7, **dadurch gekennzeichnet, dass** sie ausgehend von dem Bakterium *Xenorhabdus nematophilus* isoliert wird.

9. Pseudopeptidische antimikrobielle Verbindung nach einem der Ansprüche 1 bis 8, **dadurch gekennzeichnet, dass** sie antifungal wirksam ist.

10. Pseudopeptidische antimikrobielle Verbindung nach Anspruch 9, **dadurch gekennzeichnet, dass** sie gegenüber phytopathogenen Pilzen wirksam ist.

11. Pseudopeptidische antimikrobielle Verbindung nach Anspruch 10, **dadurch gekennzeichnet, dass** sie gegen die Pilze der Gattung *Botrytis*, insbesondere *B. cinerea*, der Gattung *Piricularia*, insbesondere *P. oryzae*, der Gattung *Helminthosporium*, insbesondere *H. teres*, der Gattung *Fusarium*, insbesondere *F. culmorum*, der Gattung *Septoria*, insbesondere *S. tritici*, der Gattung *Alternaria*, insbesondere *A. brassicae*, der Gattung *Rhizoctonia*, insbesondere *R. solani*, der Gattung *Phytophthora* und der Gattung *Cladosporium* wirksam ist.

12. Pseudopeptidische antimikrobielle Verbindung nach Anspruch 9, **dadurch gekennzeichnet, dass** sie gegenüber für den Menschen pathogenen Pilzen wirksam ist.

13. Pseudopeptidische antimikrobielle Verbindung nach Anspruch 12, **dadurch gekennzeichnet, dass** sie gegen Pilze der Gattung *Candida* wirksam ist.

14. Pseudopeptidische antimikrobielle Verbindung nach einem der Ansprüche 1 bis 8, **dadurch gekennzeichnet, dass** sie antibakteriell wirksam ist.

15. Pseudopeptidische antimikrobielle Verbindung nach Anspruch 14, **dadurch gekennzeichnet, dass** sie gegenüber Bakterien der Gattung *Escherichia*, insbesondere *E. coli*, der Gattung *Salmonella*, der Gattung *Klebsiella*, insbesondere *K. pneumoniae*, der Gattung *Proteus*, insbesondere *P*. *vulgaris*, der Gattung *Serratia*, insbesondere *S*. *entomophila*, der Gattung *Staphylococcus*, insbesondere *S. epidermis*, der Gattung *Streptococcus*, der Gattung *Pseudomonas,* insbesondere *P. fluorescens*, der Gattung *Bacillus*, insbesondere *B. megaterium*, wirksam ist.

16. Pseudopeptidische antimikrobielle Verbindung nach Anspruch 14, **dadurch gekennzeichnet**, das sie gegenüber nosokomialen Bakterien wirksam ist.

17. Pseudopeptidische antimikrobielle Verbindung nach Anspruch 16, **dadurch gekennzeichnet, dass** sie gegenüber Bakterien der Gattung *Staphylococcus*, insbesondere *S. aureus*, der Gattung *Pseudomonas*, insbesondere *P. aeruginosa,* der Gattung *Enterococcus,* der Gattung *Enterobacter*, insbesondere *E. aerogenes*, oder der Gattung *Stenotrophomonas*, insbesondere *S. maltophila*, wirksam ist.

18. Pseudopeptidische antimikrobielle Verbindung nach einem der Ansprüche 1 bis 8, **dadurch gekennzeichnet, dass** sie antiviral wirksam ist.

19. Pseudopeptidische antimikrobielle Verbindung nach Anspruch 18, **dadurch gekennzeichnet, dass** sie gegenüber verkapselten Viren wirksam ist.

20. Pseudopeptidische antimikrobielle Verbindung nach Anspruch 19, **dadurch gekennzeichnet, dass** sie gegenüber Phagen wirksam ist.

21. Pseudopeptidische antimikrobielle Verbindung nach Anspruch 20, **dadurch gekennzeichnet, dass** sie gegenüber dem Phagen lambda wirksam ist.

22. Verfahren zur Herstellung einer pseudopeptidischen antimikrobiellen Verbindung nach einem der Ansprüche 1 bis 21, **dadurch gekennzeichnet, dass** es die Schritte umfasst:
(a) Kultivierung eines Bakteriums der Gattung *Xenorhabdus* bis zum Erreichen der stationären Kulturphase,
(b) Isolierung des Kulturüberstands,
(c) Auftrennung der in dem Überstand enthaltenen Verbindungen durch Hindurchleiten des Überstands durch wenigstens eine Chromatographiesäule,
(d) selektive Isolierung von wenigstens einer Verbindung nach einem der Ansprüche 1 bis 21 durch Isolierung der Fraktionen aus der Elution entsprechend den Chromatographie-Peaks.

23. Verfahren nach Anspruch 22, **dadurch gekennzeichnet, dass** das Bakterium der Gattung *Xenorhabdus* ein Bakterium der Spezies *Xenorhabdus nematophilus* ist.

24. Verfahren nach einem der Ansprüche 22 oder 23, **dadurch gekennzeichnet, dass** die Isolierung des Kulturüberstands durch Zentrifugation der Kultur erfolgt.

25. Verfahren nach einem der Ansprüche 22 oder 23, **dadurch gekennzeichnet, dass** die Isolierung des Kulturüberstands durch Filtration der Kultur erfolgt.

26. Agrochemische Zusammensetzung, **dadurch gekennzeichnet, dass** sie eine wirksame Menge einer Verbindung nach einem der Ansprüche 1 bis 21 oder von einem von deren in der Landwirtschaft annehmbaren Salzen oder eines Metall- oder Metalloidkomplexes von dieser Verbindung, welcher gleichfalls in der Landwirtschaft annehmbar ist, umfasst.

27. Agrochemische Zusammensetzung nach Anspruch 26, **dadurch gekennzeichnet, dass** sie außerdem wenigstens einen inerten Träger und/oder wenigstens ein grenzflächenaktives Mittel umfasst.

28. Agrochemische Zusammensetzung nach einem der Ansprüche 26 oder 27, **dadurch gekennzeichnet, dass** sie für präventive oder heilende Behandlungen gegen Krankheiten von Pflanzen, welche durch phytopathogene mikrobielle Agentien verursacht werden, eingesetzt wird.

29. Agrochemische Zusammensetzung nach Anspruch 28, **dadurch gekennzeichnet, dass** die Krankheiten durch phytopathogene Pilze verursacht werden.

30. Agrochemische Zusammensetzung nach Anspruch 28, **dadurch gekennzeichnet, dass** die Krankheiten durch phytopathogene Bakterien verursacht werden.

31. Agrochemische Zusammensetzung nach Anspruch 28, **dadurch gekennzeichnet, dass** die Krankheiten durch phytopathogene Viren verursacht werden.

32. Agrochemische Zusammensetzung nach einem der Ansprüche 26 bis 31, **dadurch gekennzeichnet, dass** sie zusätzlich zu der pseudopeptidischen Verbindung der Formel (I) wenigstens einen anderen Wirkstoff enthält.

33. Agrochemische Zusammensetzung nach Anspruch 32, **dadurch gekennzeichnet, dass** der zusätzliche Wirkstoff eine fungizide Verbindung ist.

34. Verfahren zur Bekämpfung der phytopathogenen mikrobiellen Agentien von Kulturen zu heilenden oder präventiven Zwecken, **dadurch gekennzeichnet, dass** eine wirksame Menge einer Zusammensetzung nach einem der Ansprüche 26 bis 33 auf die Samen, die Blätter oder die Stämme von Pflanzen oder die Oberflächen, insbesondere die Böden, wo diese Pflanzen wachsen oder wachsen können, aufgebracht wird.

35. Verfahren zum Schutz von Vermehrungsprodukten oder -material von Pflanzen wie auch der daraus resultierenden Pflanzen gegen durch mikrobielle Agentien hervorgerufene Krankheiten zu heilenden oder präventiven Zwecken, **dadurch gekennzeichnet, dass** man die Produkte bzw. das Material mit einer wirksamen und nicht phytotoxischen Dosis einer Zusammensetzung nach einem der Ansprüche 26 bis 33 bedeckt.

36. Verfahren nach einem der Ansprüche 34 oder 35, **dadurch gekennzeichnet, dass** die Pflanzen Kulturpflanzen sind.

37. Verfahren nach Anspruch 36, **dadurch gekennzeichnet, dass** die Kulturpflanzen genetisch transformierte Pflanzen sind.

38. Verfahren nach einem der Ansprüche 36 oder 37, **dadurch gekennzeichnet, dass** die Kulturpflanzen Getreide, Leguminosen, Obstbäume, Waldbäume, Wein, Ölfrüchtekulturen, Gemüsekulturen, Nachtschattengewächse oder Rüben sind.

39. Verfahren nach Anspruch 38, **dadurch gekennzeichnet, dass** die Getreide Weizen, Mais, Reis, Gerste, Hafer sind.

40. Pharmazeutische oder veterinärmedizinische Zusammensetzung, **dadurch gekennzeichnet, dass** sie wenigstens eine wirksame Menge einer Verbindung nach einem der Ansprüche 1 bis 21 oder von einem von deren physiologisch annehmbaren Salzen oder eines Metall- oder Metalloidkomplexes von dieser Verbindung, welcher gleichfalls physiologisch annehmbar ist, umfasst.

41. Pharmazeutische oder veterinärmedizinische Zusammensetzung nach Anspruch 40, **dadurch gekennzeichnet, dass** sie außerdem wenigstens einen inerten Träger und/oder wenigstens ein Verdünnungsmittel oder Vehikel umfasst.

42. Pharmazeutische oder veterinärmedizinische Zusammensetzung nach einem der Ansprüche 40 oder 41, **dadurch gekennzeichnet**, das sie für präventive oder heilende Behandlungen gegen Krankheiten bei Menschen oder Tieren, welche durch pathogene mikrobielle Agentien verursacht werden, eingesetzt wird.

43. Pharmazeutische oder veterinärmedizinische Zusammensetzung nach Anspruch 42, **dadurch gekennzeichnet, dass** sie zusätzlich zu der pseudopeptidischen Verbindung der Formel (I) wenigstens einen anderen Wirkstoff, welcher durch eine antimikrobielle Verbindung und/oder eine Verbindung mit therapeutischer Wirkung repräsentiert wird, umfasst.

44. Verbindung nach einem der Ansprüche 1 bis 21 für eine Verwendung als Arzneimittel bei der Behandlung oder Verhütung von Infektionskrankheiten.

## Claims

1. Antimicrobial pseudopeptide compound, **characterized in that** it has the formula (I): 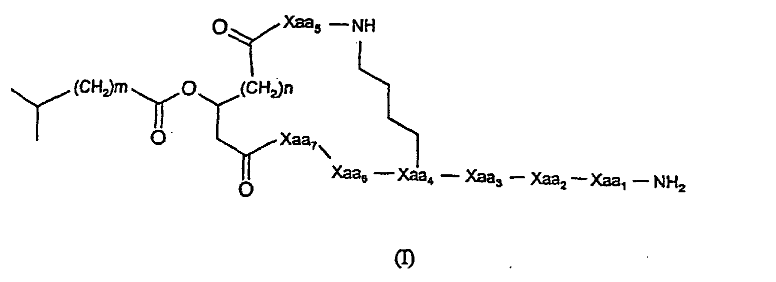 in which:
- Xaa (1 to 6) is a basic amino acid residue, preferably lysine or arginine,
- Xaa7 is glycine or an amino acid residue with an aliphatic side chain,
- m = 1 or 2 and n = 2 or 3.

2. Antimicrobial pseudopeptide compound according to Claim 1, **characterized in that** residues Xaa 1 to 6 are lysine residues.

3. Antimicrobial pseudopeptide compound according to Claim 1, **characterized in that** residues Xaa 1, 2, 3, 4 and 5 are lysine residues, and residue Xaa 6 is an arginine residue.

4. Antimicrobial pseudopeptide compound according to one of Claims 1 to 3, **characterized in that** residue Xaa 7 is a glycine residue.

5. Antimicrobial pseudopeptide compound according to one of Claims 1 to 4, **characterized in that** m is equal to 1 and n is equal to 3.

6. Antimicrobial pseudopeptide compound according to one of Claims 1 to 4, **characterized in that** m is equal to 2 and n is equal to 2.

7. Antimicrobial pseudopeptide compound according to one of Claims 1 to 6, **characterized in that** it is isolated from a bacterium of the genus *Xenorhabdus*.

8. Antimicrobial pseudopeptide compound according to Claim 7, **characterized in that** it is isolated from the bacterium *Xenorhabdus nematophilus*.

9. Antimicrobial pseudopeptide compound according to one of Claims 1 to 8, **characterized in that** it is antifungal.

10. Antimicrobial pseudopeptide compound according to Claim 9, **characterized in that** it is active against phytopathogenic fungi.

11. Antimicrobial pseudopeptide compound according to Claim 10, **characterized in that** it is active against fungi of the genus *Botrytis*, in particular *B. cinerea*, of the genus *Piricularia*, in particular *P. oryzae*, of the genus *Helminthosporium*, in particular *H. teres*, of the genus *Fusarium*, in particular *F. culmorum*, of the genus *Septoria*, in particular *S. tritici*, of the genus *Alternaria*, in particular *A. brassicae*, of the genus *Rhizoctonia,* in particular *R. solani*, of the genus *Phytophtora*, and of the genus *Cladosporium*.

12. Antimicrobial pseudopeptide compound according to Claim 9, **characterized in that** it is active against fungi which are pathogenic for humans.

13. Antimicrobial pseudopeptide compound according to Claim 12, **characterized in that** it is active against fungi of the genus *Candida*.

14. Antimicrobial pseudopeptide compound according to one of Claims 1 to 8, **characterized in that** it is antibacterial.

15. Antimicrobial pseudopeptide compound according to Claim 14, **characterized in that** it is active against bacteria of the genus *Escherichia*, in particular *E. coli*, of the genus *Salmonella*, of the genus *Klebsiella*, in particular of *K. pneumoniae*, of the genus *Proteus*, in particular *P. vulgaris*, of the genus *Serratia*, in particular *S. entomophila*, of the genus *Staphylococcus*, in particular *S. epidermis*, of the genus *Streptococcus*, of the genus *Pseudomonas*, in particular *P. fluorescens*, and of the genus *Bacillus*, in particular *B. megaterium*.

16. Antimicrobial pseudopeptide compound according to Claim 14, **characterized in that** it is active against nosocomial bacteria.

17. Antimicrobial pseudopeptide compound according to Claim 16, **characterized in that** it is active against bacteria of the genus *Staphylococcus*, in particular *S. aureus,* of the genus *Pseudomonas*, in particular *P. aeruginosa*, of the genus *Enterococcus*, of the genus *Enterobacter*, in particular *E. aerogenes*, or of the genus *Stenotrophomonas*, in particular *S. maltophila.*

18. Antimicrobial pseudopeptide compound according to one of Claims 1 to 8, **characterized in that** it is antiviral.

19. Antimicrobial pseudopeptide compound according to Claim 18, **characterized in that** it is active against encapsidated viruses.

20. Antimicrobial pseudopeptide compound according to Claim 19, **characterized in that** it is active against phages.

21. Antimicrobial pseudopeptide compound according to Claim 20, **characterized in that** it is active against the lambda phage.

22. Method for producing an antimicrobial pseudopeptide compound according to one of Claims 1 to 21, **characterized in that** it comprises the steps of:
(a) culturing a bacterium of the genus *Xenorhabdus* until the stationary culture phase is reached,
(b) isolating the culture supernatant,
(c) separating the compounds contained in the supernatant by passing said supernatant through at least one chromatography column,
(d) selectively isolating at least one compound according to one of Claims 1 to 21 by isolating the elution fractions corresponding to the chromatography peaks.

23. Method according to Claim 22, **characterized in that** the bacterium of the genus *Xenorhabdus* is a bacterium of the species *Xenorhabdus nematophilus*.

24. Method according to either of Claims 22 and 23, **characterized in that** the culture supernatant is isolated by centrifugation of said culture.

25. Method according to either of Claims 22 and 23, **characterized in that** the culture supernatant is isolated by filtration of said culture.

26. Agrochemical composition, **characterized in that** it comprises an effective amount of a compound according to one of Claims 1 to 21 or one of its agriculturally acceptable salts, or a metal or metalloid complex of this compound which is also agriculturally acceptable.

27. Agrochemical composition according to Claim 26, **characterized in that** it also comprises at least one inert support and/or at least one surfactant.

28. Agrochemical composition according to either of Claims 26 and 27, **characterized in that** it is used for preventive or curative treatments against plant diseases caused by phytopathogenic microbial agents.

29. Agrochemical composition according to Claim 28, **characterized in that** the diseases are caused by phytopathogenic fungi.

30. Agrochemical composition according to Claim 28, **characterized in that** the diseases are caused by phytopathogenic bacteria.

31. Agrochemical composition according to Claim 28, **characterized in that** the diseases are caused by phytopathogenic viruses.

32. Agrochemical composition according to one of Claims 26 to 31, **characterized in that** it contains, in addition to the pseudopeptide compound of formula (I), at least one other active material.

33. Agrochemical composition according to Claim 32, **characterized in that** the additional active material is a fungicidal compound.

34. Method for curatively or preventively controlling phytopathogenic microbial agents of crops, **characterized in that** an effective amount of a composition according to one of Claims 26 to 33 is applied to the seeds, the leaves or the trunks of plants, or the surfaces, in particular the soils, where these plants grow or are likely to grow.

35. Method for preventively or curatively protecting plant multiplication products, and also the plants resulting therefrom, against diseases caused by microbial agents, **characterized in that** said products are coated with an effective and non-phytotoxic dose of a composition according to one of Claims 26 to 33.

36. Method according to either of Claims 34 and 35, **characterized in that** the plants are crop plants.

37. Method according to Claim 36, **characterized in that** the crop plants are genetically transformed plants.

38. Method according to either of Claims 36 and 37, **characterized in that** the crop plants are cereals, leguminous plants, fruit trees, woodland trees, grape vine, oil-yielding crops, market garden crops, solanaceous plants or beetroot.

39. Method according to Claim 38, **characterized in that** the cereals are wheat, maize, rice, barley or oats.

40. Pharmaceutical or veterinary composition, **characterized in that** it comprises at least an effective amount of a composition according to one of Claims 1 to 21 or one of its physiologically acceptable salts, or a metal or metalloid complex of this compound which is also physiologically acceptable.

41. Pharmaceutical or veterinary composition according to Claim 40, **characterized in that** it also comprises at least one inert support and/or at least one diluent or excipient.

42. Pharmaceutical or veterinary composition according to either of Claims 40 and 41, **characterized in that** it is used for preventive or curative treatments against human or animal diseases caused by pathogenic microbial agents.

43. Pharmaceutical or veterinary composition according to Claim 42, **characterized in that** it comprises, in addition to the pseudopeptide compound of formula (I), at least one other active material represented by an antimicrobial compound and/or a compound having a therapeutic effect.

44. Compound according to one of Claims 1 to 21, for use as a medicinal product in the treatment or prevention of infectious diseases.
